# EUROPÄISCHE PATENTANMELDUNG

(11) **EP 3 103 479 A1**
(43) Veröffentlichungstag der Anmeldung: **14.12.2016**
(21) Anmeldenummer: 16001243.1
(22) Anmeldetag: 02.06.2016
(51) Int. Cl.: A61L 2/22, A61G 9/02, A61L 2/24, B08B 3/02, E03C 1/04

(54) **REINIGUNGSVORRICHTUNG, INSBESONDERE DESINFEKTIONSVORRICHTUNG FÜR EINE BRAUSE**

(30) Priorität: 03.06.2015 DE 102015108779
(71) Anmelder: Schneider, Hartmut J., 67126 Hochdorf-Assenheim (DE)
(72) Erfinder: Schneider, Hartmut J., 67126 Hochdorf-Assenheim (DE)
(74) Vertreter: Patentanwälte Dr. Keller, Schwertfeger

(57) **Zusammenfassung**

Die vorliegende Erfindung betrifft eine Reinigungsvorrichtung umfassend einen berührungslos betätigbaren Dosierspender (12; 112; 312) mit einem Applikationskopf (14; 114; 314) zur Ausgabe eines Reinigungsfluids, eine Brausenhalterung (18; 118; 318; 418; 518) und einen Aufnahmebereich (22; 122; 322; 522) zur Aufnahme wenigstens eines Abschnitts einer Brause (20; 120; 320; 420; 520), wobei der Dosierspender (12; 112; 312) ausgelegt ist, ein Reinigungsfluid in den Aufnahmebereich (22; 122; 322; 522) zur Aufnahme wenigstens eines Abschnitts einer Brause (20; 120; 320; 420; 520) einzutragen.

## Beschreibung

Die vorliegende Erfindung betrifft eine Reinigungsvorrichtung, insbesondere eine Desinfektionsvorrichtung für eine Brause.

Es ist bekannt, dass Infektionskrankheiten, die von Bakterien oder Viren verursacht werden, u.a. über Kotpartikel übertragen werden können. Eine solche Übertragung von Kotpartikeln von einer Person auf die andere kann vor allem bei einem Toilettengang stattfinden.

Im Kulturbereich des Islam erfordern religiös vorgeschriebene Reinigungszeremonien die Reinigung von Genitalien und Analbereich nach einem Toilettenaufenthalt. Hierfür sind in unmittelbarer Nähe der Toiletten Brausen vorgesehen, um den erforderlichen Reinigungsvorgang durchzuführen.

Da es nicht ausbleibt, dass die Brause selbst beim Reinigungsvorgang verschmutzt wird, gelangen Schmutzpartikel und Krankheitserreger an die Brause. Der nachfolgende Toilettenbesucher, der die verschmutzte Brause ergreift, kann somit über die Hände die an der Brause anhaftenden Schmutzpartikel und gegebenenfalls die darin enthaltenen Krankheitserreger aufnehmen, Handelt es sich bei den Schmutzpartikeln um pathogene Erreger, kann dies zu einer Erkrankung des nachfolgenden Toilettenbesuchers führen.

Es ist Aufgabe der vorliegenden Erfindung, eine Vorrichtung, insbesondere eine Reinigungsvorrichtung für Brausen bereitzustellen, die eine Übertragung von pathogenen Erregern von einem Toilettenbesucher zum anderen verhindert.

Erfindungsgemäß wird die Aufgabe durch eine Reinigungsvorrichtung gelöst, umfassend einen berührungslos betätigbaren Dosierspender mit einem Applikationskopf zur Ausgabe eines Reinigungsfluids, eine Brausenhalterung und einen Aufnahmebereich zur Aufnahme wenigstens eines Abschnitts einer Brause, wobei der Dosierspender ausgelegt ist, ein Reinigungsfluid in den Aufnahmebereich zur Aufnahme wenigstens eines Abschnitts einer Brause einzutragen.

Diese Reinigungsvorrichtung, die die Aufnahme einer Brause, insbesondere eines Brausenkopfs und Brausengriffs vorsieht, ermöglicht die automatische Reinigung der Brause mittels eines Reinigungsfluids.

Unter Reinigungsfluid wird jedes Mittel verstanden, das geeignet ist, die Brause zu säubern und/oder zu desinfizieren.

Vorteilhafterwelse ist das Reinigungsfluid ein Desinfektionsmittel.

Das Reinigungsfluid bzw. Desinfektionsmittel ist vorzugsweise ein Gas oder ein Aerosol. Ein Gas oder ein Aerosol hat den Vorteil, die Brause in Form einer Gas- oder Aerosolwolke zu umgeben, so dass die Brause, auch wenn sie einseitig besprüht wird, von allen Seiten gereinigt, insbesondere desinfiziert wird. Es treten hierbei vergleichsweise wenige Sterischatten auf.

Bei einer bevorzugten Ausführungsform ist ein Diffuser vorgesehen, der den Aufnahmebereich zur Aufnahme wenigstens eines Abschnitts einer Brause zumindest abschnittsweise umgibt.

Der Diffuser, der das Reinigungsfluid aus dem Dosierspender verteilt, trägt dazu bei, dass das Reinigungsfluid an möglichst viele Abschnitte der Brause gelangt.

Hierbei ist bevorzugt, dass der Diffuser gekrümmt oder gewinkelt ausgebildet ist, um einen möglichst großen Abschnitt des Aufnahmebereichs zu bedecken. Auf diese Weise können möglichst viele Stellen der Brause gereinigt werden.

Bei einer bevorzugten Weiterbildung ist ein erster Sensor, vorzugsweise ein optischer Sensor vorgesehen, der ein Signal liefert, um die Ausgabe des Reinigungsfluids auszulösen. Hierdurch ist eine berührungslose Reinigung der Brause möglich.

Um eine Verunreinigung der Umgebung aufgrund von spritzendem Reinigungsfluid zu vermeiden, ist es von Vorteil, dass ein Gehäuse vorgesehen ist und zumindest der Aufnahmebereich zur Aufnahme wenigstens eines Abschnitts einer Brause, der Applikationskopf zur Ausgabe eines Reinigungsfluids und/oder ein Diffuser in dem Gehäuse angeordnet sind.

Für eine einfache Entnahme der Brause aus dem Gehäuse ist es von Vorteil, dass das Gehäuse eine Öffnung aufweist.

Bei einer bevorzugten Weiterbildung ist eine Blende vorgesehen, die ausgelegt ist, die Öffnung zu verschließen, um zu vermeiden, dass Reinigungsfluid während des Reinigungsvorgangs durch die Öffnung des Gehäuses nach außen gelangt.

Es ist weiterhin von Vorteil, dass die Blende automatisch öffenbar ist, um eine Kontamination des Gehäuses mit pathogenen Erregern zu verhindern.

Beispielsweise ist bei einer vorteilhaften Weiterbildung ein zweiter Sensor vorgesehen, der ein Signal liefert, um die Blende automatisch zu öffnen, so dass keine Berührung des Gehäuses oder der Blende stattfindet und somit eine Verunreinigung des Gehäuses oder der Blende verhindert wird.

Um zu verhindern, dass Reinigungsflüssigkeit entlang des Brausenschlauchs nach unten läuft und gegebenenfalls auf den Boden tropft, ist in der Halterung zur Aufnahme der Brause ein Tropffänger, insbesondere eine Tropfenbürste vorgesehen.

Bevorzugte Ausführungsformen der Erfindung werden anhand der beigefügten Zeichnungen näher erläutert, in denen zeigen:
- Fig. 1: eine Seitenansicht einer ersten Ausführungsform einer Reinigungsvorrichtung, wobei eine Gehäuseseite weggelassen ist,
- Fig. 2: eine Vorderansicht der Reinigungsvorrichtung ohne Blende,
- Fig. 3: einen Querschnitt durch eine Seitenansicht der zweiten Ausführungsform einer Reinigungsvorrichtung,
- Fig. 4: einen Diffuser gemäß einer ersten Ausführungsform,
- Fig. 5: einen Diffuser gemäß einer zweiten Ausführungsform, und
- Fig. 6: eine Aufnahme eines Dosierspenders,
- Fig. 7: eine Vorderansicht einer dritten Ausführungsform einer Reinigungsvorrichtung,
- Fig. 8: eine Vorderansicht einer vierten Ausführungsform einer Reinigungsvorrichtung,
- Fig. 9: eine Vorderansicht einer fünften Ausführungsform einer Reinigungsvorrichtung in Grundstellung,
- Fig. 10: eine Brausenhalterung der fünften Ausführungsform einer Reinigungsvorrichtung,
- Fig. 11: die in Fig. 9 dargestellte Reinigungsvorrichtung in einer Desinfektions-/Reinigungsstellung und
- Fig. 12: die in Fig. 9 dargestellte Reinigungsvorrichtung in einer Servicestellung.

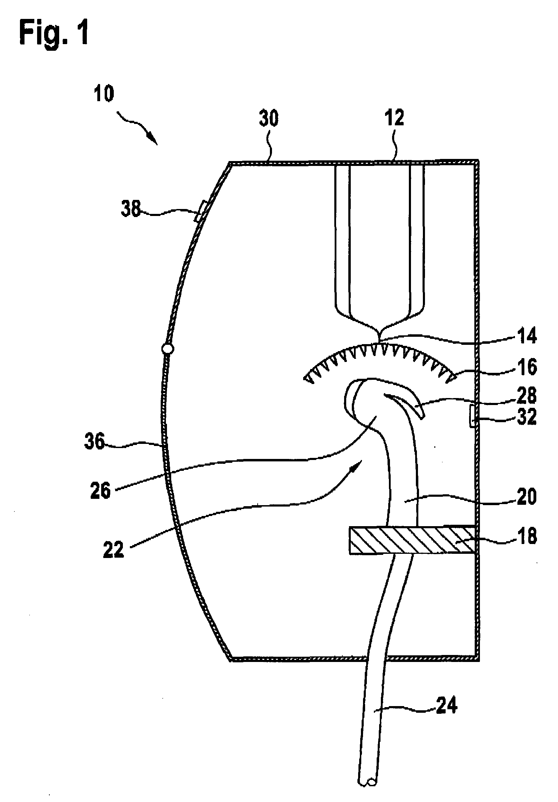

Fig. 1 zeigt eine Reinigungsvorrichtung 10 für eine Brause in einer Seitenansicht.

Die Reinigungsvorrichtung 10 umfasst einen berührungslos betätigbaren Dosierspender 12 mit einem Applikationskopf 14 zur Ausgabe eines Reinigungsfluids, einen Diffuser 16 und eine Halterung 18 zur Aufnahme einer Brause 20.

Zwischen dem Diffuser 16 und der Brausenhalterung 18 ist ein Aufnahmebereich 22 zur Aufnahme der Brause 20 vorgesehen.

Der Diffuser 16 umgibt den Aufnahmebereich 22 für die Brause 20 zumindest abschnittsweise. Die Brause 20 ist mit einem Schlauch 24 zur Versorgung mit Wasser verbunden und weist einen Brausenkopf 26 und einen Brausengriff 28 auf.

Bei der dargestellten Ausführungsform sind sowohl der Brausenkopf 26 als auch der Brausengriff 28 der Brause 20 in dem Aufnahmebereich 22 zwischen Diffuser 16 und Halterung 18 angeordnet.

Bei nicht dargestellten Ausführungsformen kann beispielsweise nur der Brausenkopf 26 oder der Brausengriff 28 oder Teile davon in dem Aufnahmebereich 22 angeordnet sein.

Entscheidend ist, dass in den Aufnahmebereich 22 die Teile der Brause gelangen, die von einem Benutzer bewusst manuell berührt wurden wie etwa der Brausengriff 28 und/oder der Brausenkopf. Darüber hinaus sollten auch die Teile der Brause gereinigt werden, mit denen der Nutzer unbewusst oder versehentlich in Berührung gelangt ist.

Der Dosierspender 12, der Diffuser 16, der Aufnahmebereich 22 zur Aufnahme einer Brause 20 und die Halterung 18 sind in einem Gehäuse 30 angeordnet.

Im Inneren des Gehäuses 30 befindet sich an einer Gehäusewand ein erster Sensor in Form eines optischen Sensors 32. Der optische Sensor 32 erfasst beispielsweise die Anwesenheit der Brause 20.

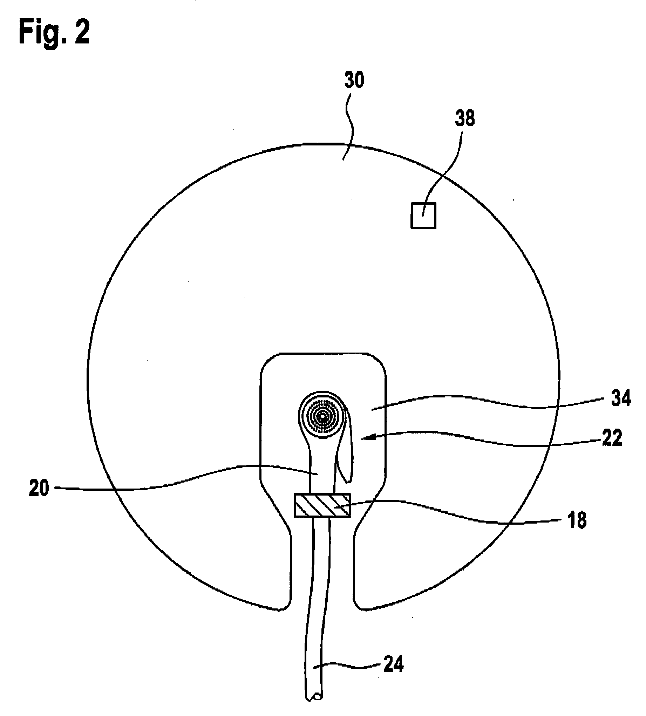

Wie insbesondere Fig. 2 zeigt, weist das Gehäuse 30 eine Öffnung 34 in Höhe des Aufnahmebereichs 22 für die Brause 20 auf. Die Öffnung 34 ist mittels einer beispielsweise schwenkbaren Blende 36 verschließbar. An der Vorderseite des Gehäuses 30 befindet sich außerhalb des Gehäuses 30 ein zweiter Sensor beispielsweise in Form eines Bewegungsmelders 38, der ein Signal liefert, die Blende 36 automatisch zu öffnen, wenn sich ein Benutzer der Reinigungsvorrichtung 10 nähert.

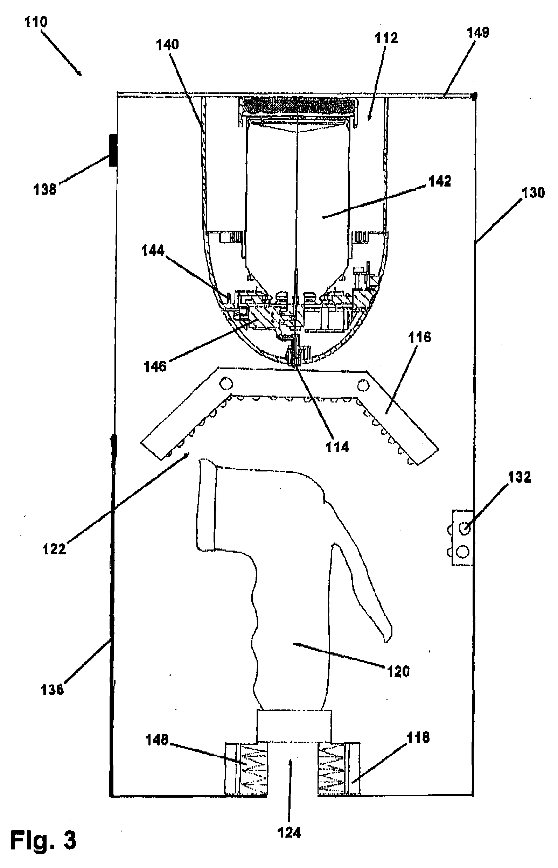

Fig. 3 zeigt eine weitere Ausführungsform einer Reinigungsvorrichtung 110 in Seitenansicht mit weiteren Details.

Der Dosierspender 112 umfasst ein Spendergehäuse 140, welches an dem Gehäuse 130 befestigt ist. In dem Spendergehäuse 140 befindet sich ein Behälter 142 mit Reinigungsfluid und eine Klemmvorrichtung 144 zur Aufnahme des Behälters 142. Das Reinigungsfluid ist ein Gas oder Aerosol. Weiterhin ist ein Magnetventil 146 vorgesehen, um den Behälter 142 in einem geöffneten oder geschlossenen Zustand zu halten.

Unterhalb des Dosierspenders 112 ist der Diffuser 116 angeordnet. Der Diffuser 116 ist abgewinkelt und umgibt den Aufnahmebereich 122 der Brause 120 oberhalb der Brause 120.

Die Brause 120 befindet sich in der Halterung 118, wobei die Halterung 118 eine Tropfbürste 148 aufweist.

An der inneren Gehäusewand des Gehäuses 130 befindet sich auf Höhe des Aufnahmebereichs der Brause 120 der optische Sensor 132.

Das Gehäuse 130 weist eine Deckplatte, Seitenwände und eine Bodenplatte auf. Die Deckplatte ist lösbar an den Seitenwänden befestigt, um die Möglichkeit zu haben, den Behälter 142 in dem Spendergehäuse 140 auszutauschen. Die vordere Seite des Gehäuses 130 ist mit einer Öffnung 134 versehen, die von einer Blende 136 bedeckt werden kann.

An der Außenseite der vorderen Seitenwand des Gehäuses befindet sich ein Bewegungsmelder 138 und in der Bodenplatte ist eine Durchführung für den Schlauch 124 vorgesehen.

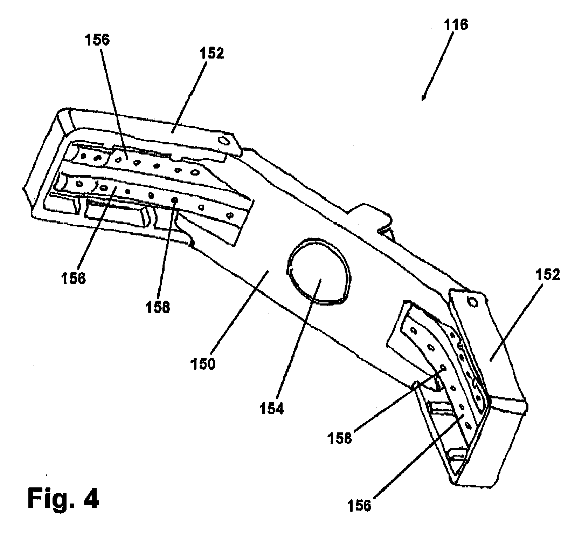

Fig. 4 zeigt eine Diffuser 116 im Detail. Der Diffuser 116 weist eine Basis 150 auf, an deren beiden Seiten jeweils ein Arm 152 schwenkbar angeordnet ist. An der Basis 150 des Diffusers 116 ist eine Öffnung 154 für den Durchtritt von Reinigungsfluid aus dem Dosierspender 112 vorgesehen. In den beiden Seitenarmen 152 sind Kanäle 156 vorgesehen, die mit dem Applikationskopf des Dosierspenders 112 in Verbindung stehen, so dass Reinigungsflüssigkeit durch die Kanäle 156 strömen kann. Die Kanäle 156 weisen eine Vielzahl von Öffnungen 158 auf, durch die die Reinigungsflüssigkeit austreten kann.

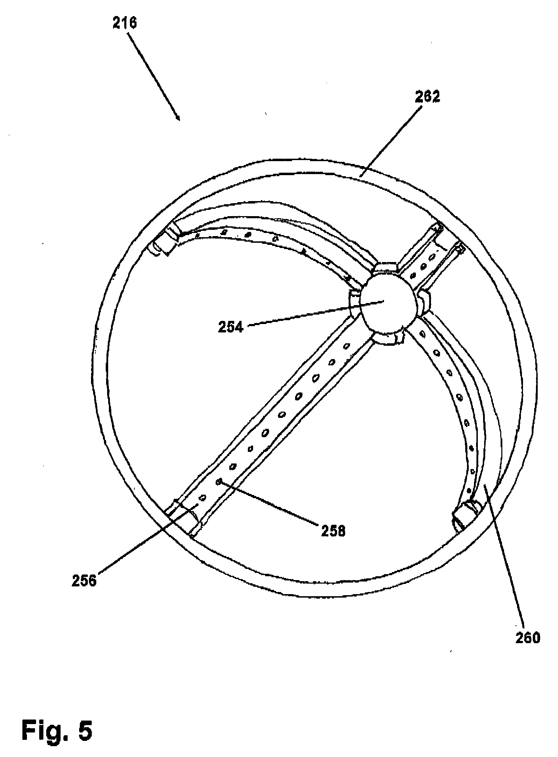

Fig. 5 zeigt eine zweite Ausführungsform eines Diffusers 216, bei dem ein Verteilerkreuz 260 vorgesehen ist. Das Verteilerkreuz 260 ist an seinen freien Enden jeweils in einer kreisförmigen Halterung 262 befestigt. Die beiden Arme des Verteilerkreuzes 260 weisen eine größere Länge als der Durchmesser der kreisförmigen Halterung 262 auf, so dass das aus einem elastischen Material bestehende Verteilerkreuz 260 sich nach außen wölbt und der Diffuser 216 die Form einer Glocke annimmt. Der Diffuser 216 weist eine Öffnung 254 für den Applikationskopf des Dosierspenders auf. In den Armen des Verteilerkreuzes sind Kanäle 256 mit Öffnungen 258 vorgesehen, durch die das Reinigungsfluid nach unten in den Aufnahmebereich und somit auf die Brause gelangen kann.

Vorzugsweise ist der Diffuser 116, 216 fest mit dem Dosierspender 112 verbunden. Im Rahmen der Erfindung ist auch die Verwendung von Diffusern und Dosierspendern möglich, die nicht miteinander verbunden sind und nur in Wirkverbindung stehen.

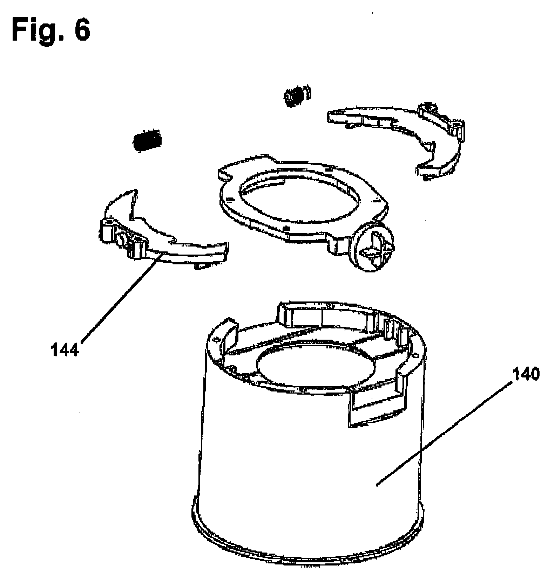

Fig. 6 zeigt ein Spendergehäuse 140 mit einer Klemmvorrichtung 144. Der Behälter 142 wird in bekannter Weise in der Klemmvorrichtung 144 gehalten.

Anstelle einer abnehmbaren Deckplatte kann das Gehäuse 130 alternativ eine Deckplatte aufweisen, die schwenkbar an einer Seitenwand des Gehäuses 130 befestigt ist.

Wenn auch nicht dargestellt so ist eine Steuerung- und/oder Regelungsvorrichtung vorgesehen, die die von den Sensoren empfangen Signale weiterleitet und die aufgrund der empfangenen Signale vorbestimmten Aktionen aktiviert.

Als Reinigungsfluid eignen sich nicht nur Gase oder Aerosole wie in der Ausführungsform oben beschreiben, sondern auch Flüssigkeiten wie etwa flüssige Seifen oder flüssige Desinfektionsmittel. Das Reinigungsfluid ist ausgelegt die Brause zu reinigen und/oder zu desinfizieren. Somit kann das Reinigungsfluid sowohl Desinfektionsmittel als auch Reinigungsmittel umfassen.

Unter Reinigungsmittel wird eine Mittel verstanden, das primär geeignet ist, Schmutzpartikel von der Brause zu entfernen, während ein Desinfektionsmittel ein Mittel ist, das primär der Abtötung von Krankheitserregern dient.

Vorzugsweise handelt es sich bei dem Reinigungsfluid um ein Desinfektionsmittel, um Bakterien, Keime oder pathogene Erreger abzutöten. Hierbei sind insbesondere Desinfektionsmittel in Form eines Gases oder als Aerosol bevorzugt, da diese eine entsprechende Gas- oder Aerosolwolke bilden, die sich auch an der Unterseite der Brause 20, 120 welche nicht in Richtung des Dosierspenders 12, 112 gerichtet ist, niederschlagen. Somit kann auch die Unterseite der Brause 20, 120 desinfiziert werden.

Um neben der Desinfektion auch eine Reinigung der Brause bereitzustellen, kann das Desinfektionsmittel mit einem Reinigungsmittel vermischt sein.

Als Dosierspender eignen sich alle Arten von berührungslos arbeitenden Dosierspendern, wie etwa unter Druck stehende Sprühdosen, die beispielsweise mittels eines Magnetventils ausgelöst werden oder auch Dosierspender, die mit Hilfe einer elektrischen Pumpe ein Fluid dosiert ausgeben.

Die Energieversorgung der Reinigungsvorrichtung und der Dosierspender erfolgt vorzugsweise über eine Batterie.

Die Inbetriebnahme und die Funktion der Reinigungsvorrichtung wird beispielhaft anhand der In Figur 3 dargestellten Ausführungsform beschrieben.

Zur Inbetriebnahme der Reinigungsvorrichtung 110 wird die Oberseite des Gehäuses 130 geöffnet und ein Behälter 142 in bekannter Weise mit dem Applikationskopf 114 nach unten in das Spendergehäuse 140 eingeführt und mittels der Klemmvorrichtung 144 lösbar im Spendergehäuse 140 befestigt. Nachdem der Behälter 142 in dem Spendergehäuse 140 befestigt wurde, wird die Deckplatte des Gehäuses 130 geschlossen. Zum Austausch eines entleerten Behälters 142 wird die Deckplatte des Gehäuses 130 wieder geöffnet und der leere Behälter gegen einen vollen Behälter ersetzt.

Im Grundzustand der Reinigungsvorrichtung 110 befindet sich die Brause 120 in ihrer Halterung 118 im Gehäuse 130. Die Blende 134 ist verschlossen. Es tritt keine Reinigungsflüssigkeit aus dem Dosierspender 112 aus.

Wird der Gebrauch der Brause 120 gewünscht, nähert sich der Nutzer der Vorrichtung 110. Mittels des Sensors 138 wird eine Bewegung des Nutzers erfasst und ein Signal an eine nicht dargestellte Steuereinheit gegeben, um die Blende 136 automatisch zu öffnen. Der Nutzer kann die Brause 120 durch die nun freigegebene Öffnung entnehmen und den gewünschten Säuberungsvorgang durchführen.

Nach Beendigung des Säuberungsvorgangs führt der Nutzer die Brause 120 durch die Öffnung in das Gehäuse 130 und hängt die Brause 120 wieder in die Halterung 118.

Der Sensor 132 im Inneren des Gehäuses 130 ist ausgelegt, die Anwesenheit der Brause 120 in der Halterung 118 und die Stellung der Blende 136 zu erfassen.

Nachdem der Sensor 132 das Signal erfasst hat, dass sich die Brause 120 in der Halterung 118 befindet, wird ein Befehl ausgegeben, die Blende 136 zu verschließen. Sobald der Sensor 132 erkennt, dass die Blende 136 verschlossen ist, wird über die nicht dargestellte Steuer- und/oder Regelungsvorrichtung ein Signal an den automatisch betätigbaren Dosierspender 112 gegeben, ein Reinigungsfluid auszugeben. Im dargestellten Ausführungsbeispiel handelt es sich um ein gasförmiges Reinigungsfluid oder um ein Reinigungsfluid in Form eines Aerosols, wobei der Behälter 142 unter Druck steht.

Sobald der Dosierspender 112 ein Signal empfangen hat, Reinigungsfluid auszugeben, wird das Magnetventil 144 betätigt, um das Reinigungsfluid auszugeben.

Der Dosierspender 112 gibt die voreingestellte Menge an Reinigungsfluid über den Applikationskopf an den Diffuser 116 aus. Der Diffuser 116 verteilt das Reinigungsfluid oberhalb der Brause 120 in dem Aufnahmebereich 122 der Brause 120. Das Reinigungsfluid schlägt sich an der Brause 120 nieder und reinigt bzw. desinfiziert die Brause 120.

Das Reinigungsfluid, welches sich an der Brause 120 niederschlägt und entlang der Brause in Richtung des Schlauches 124 läuft, wird mittels der Tropfbürste 148 in der Halterung 118 zurückgehalten. Das in der Tropfbürste 148 gesammelte Reinigungsfluid, kann dann bei geeigneter Wahl des Reinigungsfluid verdunsten. Es entstehen keine unerwünschten Tropfflecken auf dem Boden unterhalb der Reinigungsvorrichtung 110.

Während des Reinigungsvorgangs der Brause 120, der Je nach Anforderung mehrere Sekunden dauern kann, bleibt das Gehäuse 130 geschlossen. Erst wenn der Reinigungsvorgang abgeschlossen ist, gelangt die Reinigungsvorrichtung in ihren Grundzustand und oben beschriebener Vorgang kann wiederholt werden, der damit beginnt, dass der Bewegungsmelder außerhalb des Gehäuses 130 ein Signal erfasst und einen Befehl liefert, die Blende 136 des Gehäuses 130 zu öffnen.

Bei einem alternativen Verfahren zur Reinigung der Brause 120 kann der Grundzustand darin bestehen, dass das Gehäuse unverschlossen und somit die Öffnung unbedeckt ist, um die gereinigte Brause zu entnehmen. Während des Reinigungs.-bzw. Desinfektionsvorgangs der Brause wird das Gehäuse automatisch verschlossen. Nach Beendigung des Reinigungs.-bzw. Desinfektionsvorgangs der Brause wird die Öffnung automatisch freigegeben, so dass die Reinigungsvorrichtung wieder in ihren Grundzustand gelangt, in dem das Gehäuse unverschlossen und die Öffnung unbedeckt ist.

Bei der dargestellten Ausführungsform ist der Dosierspender oberhalb der Brause angeordnet, bei einer nicht dargestellten Ausführungsform ist der Dosierspender seitlich oder unterhalb der Brause angeordnet. Entscheidend ist, dass der Dosierspender so ausgerichtet ist, dass das über den Applikationskopf abgegebene Reinigungsfluid in den Aufnahmebereich zur Aufnahme der Brause gelangt.

Anstelle eines im Wesentlichen geschlossenen Gehäuses mit einer von einer Blende bedeckbaren Öffnung und einer Durchführung für den Schlauch der Brause, kann im Rahmen der Erfindung ein Gehäuse vorgesehen sein, das an der Ober- und/oder Unterseite offen ist. Zusätzlich oder alternativ können einzelne Seitenwände weggelassen sein.

Bei einer nicht dargestellten Ausführungsform können die Halterung für die Brause, der Dosierspender und/oder der Diffuser einzeln beispielsweise an einer Wand befestigt sein, so dass bei Bedarf das Gehäuse weggelassen werden kann.

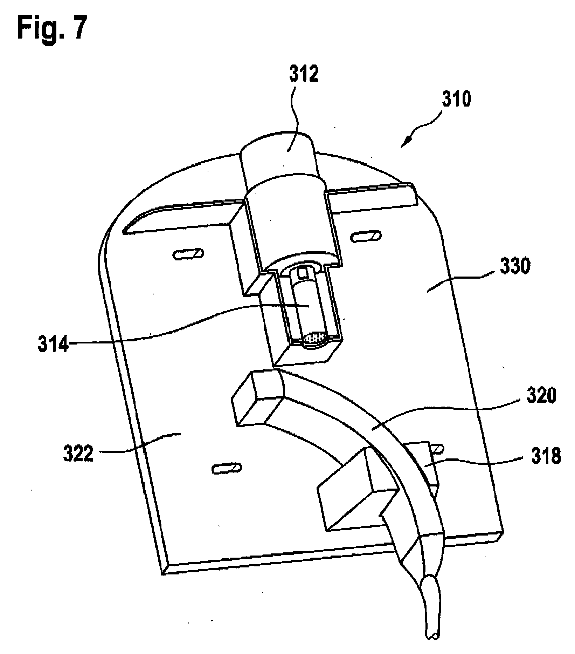

Fig. 7 zeigt eine dritte Ausführungsform 310 einer Reinigungsvorrichtung, bei der der Diffuser und ein Teil des Gehäuses 330 weggelassen sind. Das Gehäuse 330 weist lediglich eine Rückwand auf, an der der Dosierspender 312 mit einem Applikationskopf 314 befestigt ist.

Weiterhin ist an dem Gehäuse 330 eine Brausenhalterung 318 vorgesehen, wobei zwischen Brausenhalterung 318 und dem Applikationskopf 314 der Aufnahmebereich 322 zur Aufnahme einer Brause 320 ausgebildet ist. In dem Dosierspender 312 befindet sich vorzugsweise ein Gas oder Aerosol eines Desinfektionsmittels. Bei dieser Ausführungsform verteilt der Applikationskopf 314 des Dosierspenders 312 das Reinigungsfluid, insbesondere das Desinfektionsmittel oberhalb der Brause 320, um zumindest einen Teil der Brause zu reinigen bzw. zu desinfizieren.

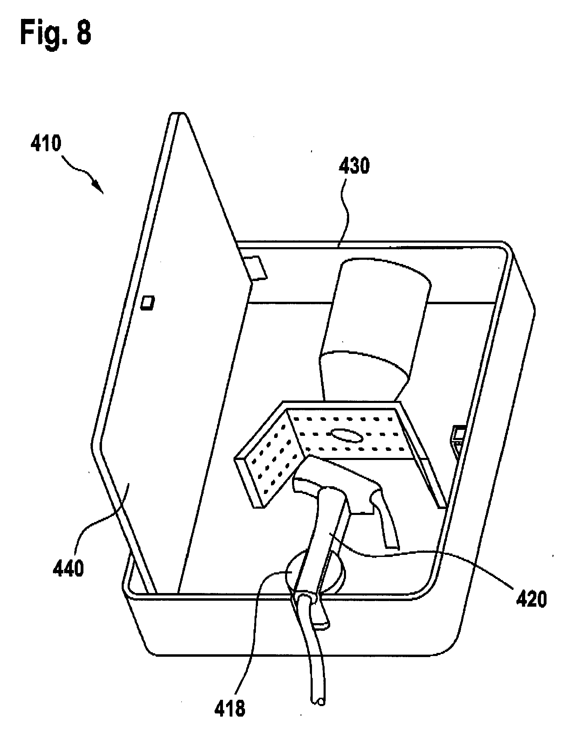

Bei der in Fig. 8 dargestellten dritten Ausführungsform 410 einer Reinigungsvorrichtung wird das Gehäuse 430 mittels einer Blende in Form einer Drehtür 440 verschlossen, wobei die gesamte Vorderseite des Gehäuses 430 die Öffnung zur Entnahme der Brause 420 bildet.

Diese Ausführungsform ist beispielsweise für eine manuelle Öffnung des Gehäuses 430 geeignet. Mittels eines nicht dargestellten Griffs oder Druckknopfs wird die Drehtür 440 geöffnet, um die Brause 420 aus dem Gehäuse 430 zu entnehmen. Nach Beendigung des gewünschten Säuberungsvorgangs mit der Brause 420 wird diese wieder in die Halterung 418 eingehängt. Die Drehtür 440 kann nun wieder manuell verschlossen werden, um die Reinigung bzw. Desinfektion der Brause 420 durchzuführen.

Alternativ ist das Verschließen des Gehäuses 430 für die Durchführung des Reinigungs- bzw. Desinfektionsvorgangs der Brause 420 auch automatisch möglich, nachdem beispielsweise ein Sensor die Anwesenheit der Brause 420 in dem Gehäuse 430 erfasst hat.

Wenn in den beschriebenen Ausführungsformen auch ein optischer Sensor vorgesehen ist, der die Anwesenheit der Brause und die Stellung der Blende erfasst, kann im Rahmen der Erfindung jede Art von Sensoren verwendet werden, die geeignet ist, die Anwesenheit der Brause und die Stellung der Blende zu erfassen. Beispielsweise ist so auch die Verwendung von ein oder mehreren Kontaktsensoren oder Drucksensoren möglich.

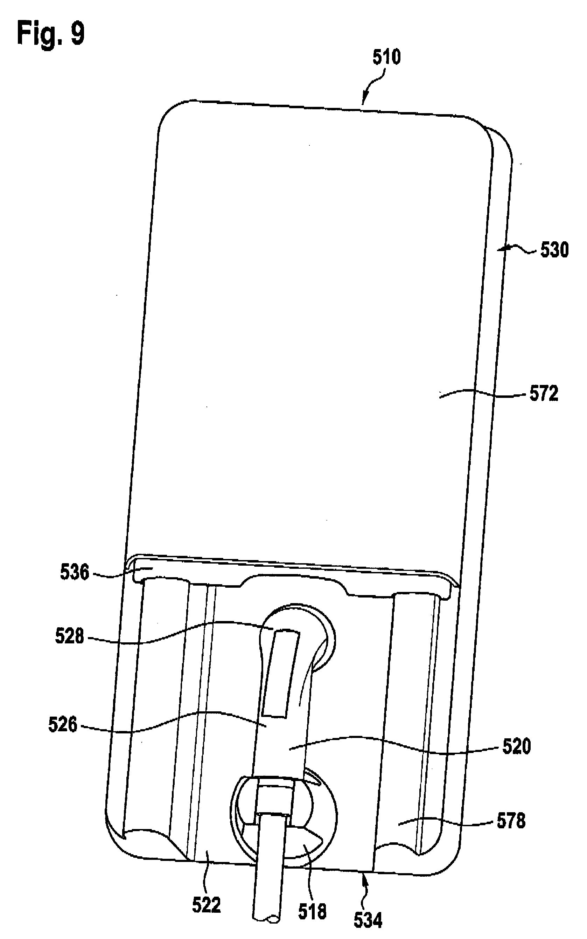

Die Figuren 9 bis 12 zeigen eine fünfte Ausführungsform einer Reinigungsvorrichtung 510.

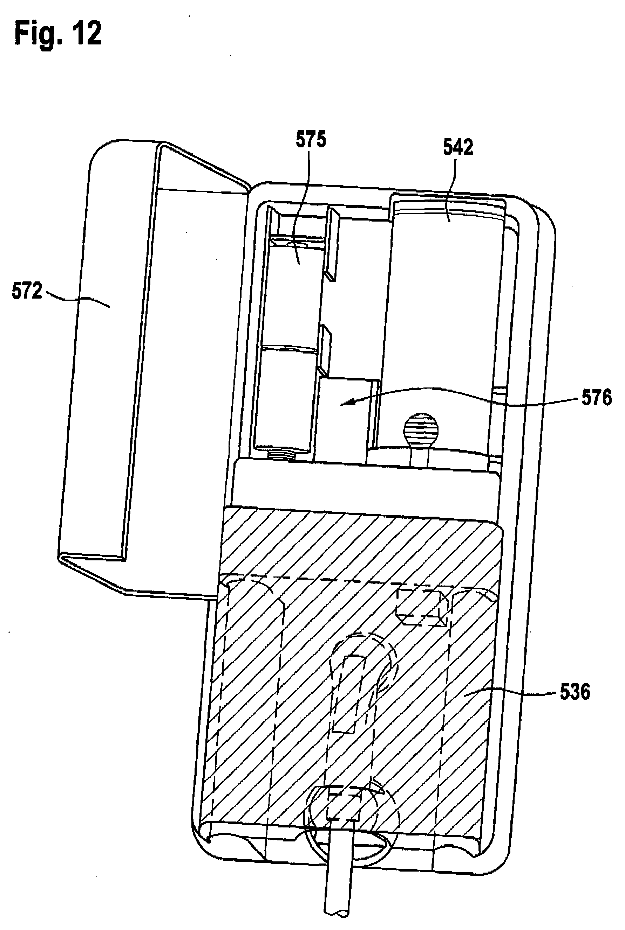

Die Reinigungsvorrichtung 510 umfasst ein Gehäuse 530, welches an der Vorderseite eine Öffnung 534 und einen Deckel 572 aufweist. Der Deckel 572 ist im oberen Abschnitt des Gehäuses 530 angeordnet und schwenkbar ausgebildet. Er bedeckt einen Behälter 542 mit einem Reinigungsfluid, sowie Batterien 575 und eine Steuervorrichtung 576 für den Betrieb der Reinigungsvorrichtung (siehe Fig. 12). Die Öffnung 534 ist im Bereich des Aufnahmebereichs 522 vorgesehen.

Die Halterung 518 zur Aufnahme der Brause 520 befindet sich auf der Innenseite des Gehäuses im Bereich der Öffnung 534, so dass die Brause 520, insbesondere der Brausengriff 526 für einen Benutzer frei zugänglich ist.

Unterhalb des Gehäusedeckels 572 ist eine Blende 536 vorgesehen, die auf Führungsleisten 578 parallel zum Deckel 572 verschiebbar ist. Die Führungsleisten 578 sind an der Vorderseite des Gehäuses 530 Im Bereich der Öffnung 534 beidseitig der Halterung 518 für die Brause 530 angebracht. Die Blende 536 ist ausgelegt, die Öffnung 534 und somit den Aufnahmebereich 522 zur Aufnahme wenigstens eines Abschnitts einer Brause in einer Reinigungsstellung zu verschließen. Im vorliegenden Ausführungsbeispiel besteht die Blende aus einem transparenten Material. Es versteht sich, dass die Blende auch aus einem nicht transparenten Material gefertigt sein kann.

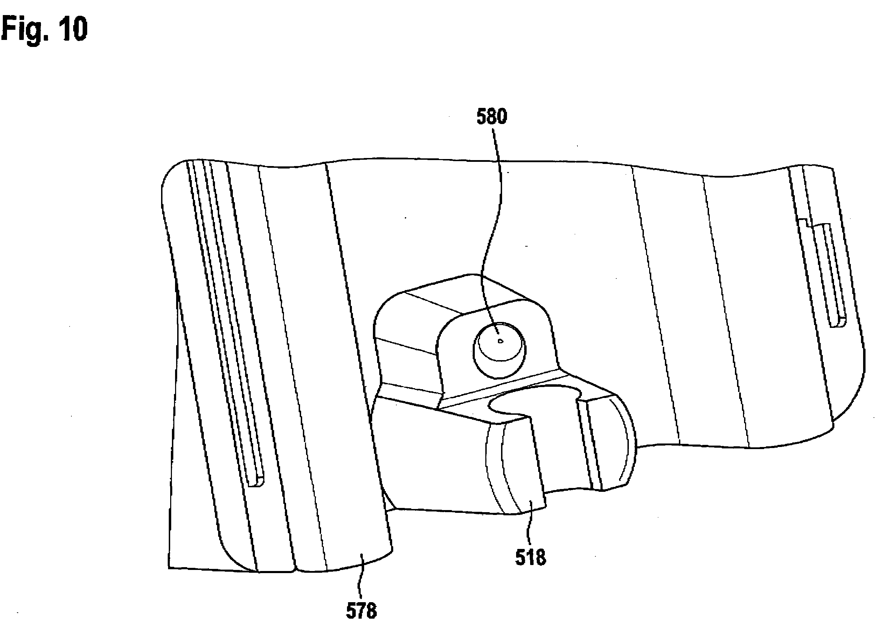

Wie insbesondere in Fig. 10 zu erkennen ist, weist die Halterung 518 einen Druckknopf 580 auf, um wie weiter unten beschrieben wird, einen Reinigungsvorgang auszulösen.

Die Fig. 9 zeigt die Reinigungsvorrichtung 510 in einer Grundstellung, In der die desinfizierte Brause 520 für den Einsatz bereit ist. In dieser Grundstellung befindet sich die Blende 536 im Wesentlichen vollständig unterhalb des Gehäusedeckels 572, so dass die Öffnung 534 frei zugänglich ist.

Ein Benutzer entnimmt die desinfizierte Brause 520 der Reinigungsvorrichtung 510 in der Grundstellung am Brausengriff 528 und führt den gewünschten persönlichen Reinigungsvorgang wie etwa die Reinigung des Genitalbereichs mit der Brause 520 durch.

Nach Beendigung des gewünschten persönlichen Reinigungsvorgangs wird die Brause 520 in die Halterung 518 gesetzt, wobei der Druckknopf 580 betätigt wird. Die Betätigung des Druckknopfs 580 löst den Reinigungs-/Desinfektionsvorgang aus, der damit beginnt, dass die Blende 536 unterhalb des Gehäusedeckels 572 entlang der Führungsleisten 578 automatisch nach unten verschoben wird, um die Öffnung 534 und somit wesentliche Teil der Brause 520, nämlich den Brausengriff 528 und den Brausenkopf 526 zu bedecken.

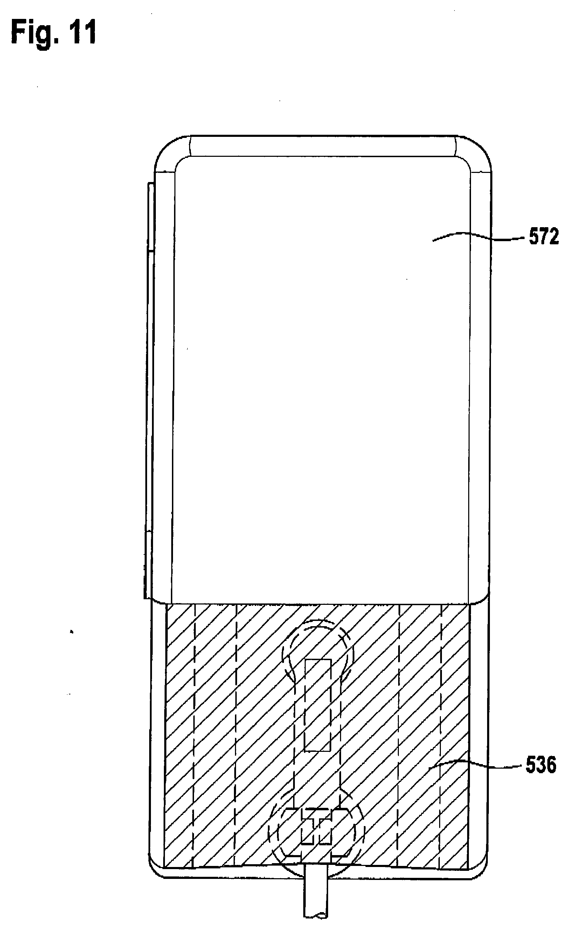

Wenn sich die Blende 536 wie in Fig. 11 dargestellt in ihrer Reinigungsstellung befindet, wird der Sprühvorgang zur Reinigung/Desinfizierung ausgelöst. Die Blende 536 sorgt dafür, dass das Desinfektionsmittel nicht an die Umgebung versprüht wird.

Nach Abschluss des Desinfektions-/Reinigungsvorgangs, die eine vorbestimmte Zeitdauer dauert, wird die Blende 536 wieder automatisch entlang der Führungsleisten 578 nach oben verschoben, bis diese im Wesentlichen vollständig unter dem Gehäusedeckel 572 verschwunden ist.

Die Reinigungsvorrichtung 510 steht nun für den nächsten Einsatz zur Verfügung.

Fig. 12 zeigt eine Servicestellung, in der ein Dosierbehälter 542 ausgetauscht werden kann. Hierbei ist die Blende 536 in eine Position verschoben, in der sie die Öffnung 534 bedeckt. Der Gehäusedeckel 572 ist zur Seite geschwenkt, sodass der Dosierbehälter 542 zugänglich wird und entnommen und ausgetauscht werden kann.

Für die Servicestellung ist ein entsprechender nicht dargestellter Verriegelungsknopf vorgesehen, der bei Bedarf betätigt werden kann, um ein versehentliches Öffnen des Gehäusedeckels 572 während des Reinigungs- bzw. Desinfektionsvorgangs zu verhindern.

Es versteht sich, dass die in Zusammenhang mit den anderen Ausführungsformen dargestellten Dosierspender und Diffuser auch bei der in den Fig. 9 bis 12 dargestellten Ausführungsform eingesetzt werden können.

Anstelle des Druckknopfes 580 kann jeder beliebige Sensor verwendet werden, der die Anwesenheit der Brause erfasst.

Weiterhin kann die in den Figuren 9 bis 12 dargestellte Ausführungsform in der Form abgewandelt werden, dass in der Grundstellung, in der eine einsatzfähige desinfizierte Brause 520 in der Reinigungsvorrichtung 510 zur Verfügung steht, die Blende 536 die Öffnung 534 bedeckt. Um die Blende 536 zu öffnen, ist dann ein Sensor vorgesehen, der einen Nutzer erfasst, welcher die Brause 520 aus der Halterung 518 entnehmen möchte. Sobald der Sensor die Anwesenheit eines Nutzers erfasst, wird die Blende 536 geöffnet.

## Patentansprüche

1. Reinigungsvorrichtung umfassend einen berührungslos betätigbaren Dosierspender (12; 112; 312) mit einem Applikationskopf (14; 114; 314) zur Ausgabe eines Reinigungsfluids, eine Brausenhalterung (18; 118; 318; 418; 518) und einen Aufnahmebereich (22; 122; 322; 522) zur Aufnahme wenigstens eines Abschnitts einer Brause (20; 120; 320; 420; 520), wobei der Dosierspender (12; 112; 312) ausgelegt ist, ein Reinigungsfluid in den Aufnahmebereich (22; 122; 322; 522) zur Aufnahme wenigstens eines Abschnitts einer Brause (20; 120; 320; 420; 520) einzutragen.

2. Reinigungsvorrichtung nach Anspruch 1 **dadurch gekennzeichnet, dass** das Reinigungsfluid ein Desinfektionsmittel ist.

3. Reinigungsvorrichtung nach einem der vorhergehenden Ansprüche, **dadurch gekennzeichnet, dass** das Reinigungsfluid ein Gas oder ein Aerosol ist.

4. Reinigungsvorrichtung nach einem der vorhergehenden Ansprüche, **dadurch gekennzeichnet, dass** ein Diffuser (16; 116; 216) vorgesehen ist, der den Aufnahmebereich (22; 122; 322) zur Aufnahme wenigstens eines Abschnitts einer Brause (20; 120; 320; 420; 520) zumindest abschnittsweise umgibt.

5. Reinigungsvorrichtung nach Anspruch 4, **dadurch gekennzeichnet, dass** der Diffuser (16; 116; 216) gekrümmt oder gewinkelt ist.

6. Reinigungsvorrichtung nach einem der vorhergehenden Ansprüche, **dadurch gekennzeichnet, dass** ein erster Sensor, vorzugsweise ein optischer Sensor (32; 132) vorgesehen ist, der ein Signal liefert, um die Ausgabe des Reinigungsfluids auszulösen.

7. Reinigungsvorrichtung nach einem der vorhergehenden Ansprüche, **dadurch gekennzeichnet, dass** ein Gehäuse (30; 130; 330; 430; 530) vorgesehen ist und zumindest der Aufnahmebereich (22; 122; 322; 522) zur Aufnahme wenigstens eines Abschnitts einer Brause (20; 120; 320; 420; 520), der Applikationskopf (14; 114; 314) zur Ausgabe eines Reinigungsfluids und/oder ein Diffuser (16; 116; 216) in dem Gehäuse (30; 130; 330; 430; 530) angeordnet sind.

8. Reinigungsvorrichtung nach einem der vorhergehenden Ansprüche, **dadurch gekennzeichnet, dass** das Gehäuse (30; 130; 330; 430; 530) eine Öffnung (34; 534) aufweist.

9. Reinigungsvorrichtung nach Anspruch 8, **dadurch gekennzeichnet, dass** eine Blende (36; 136; 536) vorgesehen ist, die ausgelegt ist, die Öffnung (34; 534) zu verschließen.

10. Reinigungsvorrichtung nach Anspruch 9, **dadurch gekennzeichnet, dass** die Blende (36; 136; 536) automatisch öffenbar ist.

11. Reinigungsvorrichtung nach Anspruch 10, **dadurch gekennzeichnet, dass** ein zweiter Sensor (38; 138) vorgesehen ist, der ein Signal liefert, um die Blende (36; 136) automatisch zu öffnen.

12. Reinigungsvorrichtung nach einem der vorhergehenden Ansprüche, **dadurch gekennzeichnet, dass** das Gehäuse einen öffenbaren Gehäusedeckel (572) sowie eine Blende (536) umfasst.

13. Reinigungsvorrichtung nach Anspruch 12, **dadurch gekennzeichnet, dass** der Gehäusedeckel (572) schwenkbar und die Blende (536) verschiebbar sind.

14. Reinigungsvorrichtung nach einem der vorhergehenden Ansprüche, **dadurch gekennzeichnet, dass** in der Halterung (116) zur Aufnahme der Brause ein Tropfenfänger, insbesondere eine Tropfbürste (148) vorgesehen ist.
